(19) Europäisches Patentamt
European Patent Office
Office européen des brevets

(11) Veröffentlichungsnummer: **0 226 023 A1**

(12) **EUROPÄISCHE PATENTANMELDUNG**

(21) Anmeldenummer: **86115312.0**

(22) Anmeldetag: **05.11.86**

(51) Int.Cl.[4]: **C 07 D 513/04**
A 61 K 31/54
//(C07D513/04, 279:00, 221:00)

(30) Priorität: **16.11.85 DE 3540702**

(43) Veröffentlichungstag der Anmeldung:
**24.06.87 Patentblatt 87/26**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Fengler, Gerd, Dr.**
**Bethelstrasse 16**
**D-4150 Krefeld 1(DE)**

(72) Erfinder: **Klausener, Alexander, Dr.**
**Dahlerdyk 173**
**D-4150 Krefeld(DE)**

(72) Erfinder: **Buysch, Hans-Josef, Dr.**
**Brandenburger Strasse 28**
**D-4150 Krefeld 1(DE)**

(72) Erfinder: **Mardin, Mithat, Dr.**
**153 Horse Pond Road**
**Madison, CT 06443(US)**

(72) Erfinder: **Pelster, Bernhard, Dr.**
**Pleisufer 6a**
**D-5205 St. Augustin 1(DE)**

(54) **1H-Pyrido-]2,3-b[]1,4[-thiazine.**

(57) 1H-Pyrido-[2,3-b][1,4]-thiazine der Formel I

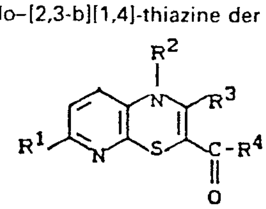

in der

$R^1$ Wasserstoff, Halogen, gegebenenfalls substituiertes Alkyl oder Alkoxy oder die Gruppe

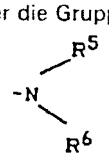

in der

$R^5$ and $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy oder die Gruppe

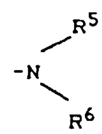

in der

$R^5$ und $R^6$ die obengenannte Bedeutung haben, oder für den Fall, daß $R^3$ Wasserstoff oder gegebenenfalls substituiertes Alkoxy bedeutet, auch gegebenenfalls substituiertes Alkoxy

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

und deren quaternäre Salze, können durch Umsetzung von entsprechenden Pyridinverbindungen mit den entsprechenden Carbonylverbindungen hergestellt werden. Die neuen Verbindungen der Formel I können als Wirkstoffe in Arzneimittel verwendet werden.

EP 0 226 023 A1

BAYER AKTIENGESELLSCHAFT 5090 Leverkusen, Bayerwerk
Konzernverwaltung RP
Patentabteilung Mn/by-c

## 1H-Pyrido-[2,3-b][1,4]-thiazine

Die vorliegende Erfindung betrifft neue 1H-Pyrido-[2,3-b][1,4]-thiazine und deren quaternäre Salze, Verfahren zu ihrer Herstellung und ihre Verwendung in Arzneimittel.

Es ist bekannt, daß die durch das Enzym Lipoxygenase gebildeten Metaboliten der Arachidonsäure an der Entstehung von entzündlichen und allergischen Prozessen beteiligt sind (E.J. Goetzl, Immunology 40, 709 (1980); Ford-Hutchinson et al., J. Pharm. Pharmacol. 32, 517 (1980) und Nature 286, 264 (1980) und Samuelsson Trends in Pharmacol. Sci., 1980, 227; Borgeat et al., J. Med. Chem. 24, 121 (1981)).

Pyrido-[2,3-b][1,4]-thiazine sind aus C.A. 83, 193384 y (1975) und C.A. 74, 141668 p (1971) bekannt.

Es wurden neue 1H-Pyrido-[2,3-b][1,4]-thiazine der Formel

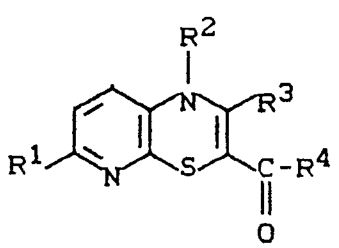

(I),

in der

$R^1$ Wasserstoff, Halogen, gegebenenfalls substituiertes Alkyl oder Alkoxy oder die Gruppe

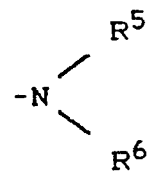

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy, die Gruppe

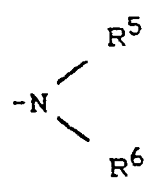

in der

$R^5$ und $R^6$ die obengenannte Bedeutung haben,

oder für den Fall, daß $R^3$ Wasserstoff oder gegebenenfalls substituiertes Alkoxy bedeutet, auch gegebenenfalls substituiertes Alkoxy bedeutet,

wobei

$R^3$ und $R^4$ über eine Polymethylenkette zu einem 5- bis 7-gliedrigen Carbocyclus verbunden sein können,

wobei im Falle eines 6-gliedrigen Carbocyclus $R^1$ nicht die Bedeutung Wasserstoff, Chlor, Methoxy oder Ethoxy hat,

und deren quaternären Salze,

gefunden.

Überraschenderweise sind die neuen 1H-Pyrido[2,3-b][1,4]-thiazine starke Lipoxygenasehemmer. Überraschenderweise hemmen sie die Lipoxygenase sehr spezifisch bereits in solchen Konzentrationen, bei denen die Cyclooxygenase nicht beeinflußt wird. Diese sehr starke und spezifische Wirkung der 1H-Pyrido[2,3-b][1,4]-thiazine konnte nicht erwartet werden.

Halogen steht im allgemeinen für Fluor, Chlor, Brom und Iod bevorzugt für Fluor, Chlor und Brom.

Alkyl steht im allgemeinen für einen geradkettigen oder verzweigten Kohlenwasserstoffrest mit 1 bis 8 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl,

Hexyl und Isohexyl genannt.

Alkoxy steht im allgemeinen für einen über ein Sauerstoffatom gebundenen, geradkettigen oder verzweigten Kohlenwasserstoffrest mit 1 bis 8 Kohlenstoffatomen. Bevorzugt ist Niederalkoxy mit 1 bis etwa 6 Kohlenstoffatomen. Beispielsweise seien Methoxy, Ethoxy, Propoxy, Isopropoxy, Butoxy, Isobutoxy, Pentoxy, Isopentoxy, Hexoxy und Isohexoxy genannt.

Aryl steht im allgemeinen für einen aromatischen Rest mit 6 bis etwa 12 Kohlenstoffatomen. Bevorzugte Arylreste sind Phenyl, Naphthyl und Diphenyl.

Die Alkyl, Alkoxy und Arylreste können substituiert sein. Beispielsweise können sie durch Halogen, bevorzugt Chlor und Brom, Hydroxy, Amino, Dimethylamino, Diethylamino, Cyano, Methoxycarbonyl oder Ethoxycarbonyl substituiert sein. Die Reste können 1 bis 3, bevorzugt einen, Substituenten tragen.

Die Reste $R_5$ und $R_6$ können zu einem 5- oder 6-gliedrigen Heterocyclus verbunden sein. Als Beispiele seien hier die folgenden Heterocyclen genannt:

Pyrrolidin, Pyrrol, Imidazol, Piperidin, Piperazin, N-Methylpiperazin und Morpholin.

Die Reste $R_3$ und $R_4$ können über eine Polymethylenkette zu einem 5- bis 7-gliedrigen Carbocyclus verbunden sein. Beispielsweise seien die folgenden Carbocyclen genannt:

Cyclopentenon, Cclohexenon und Cycloheptenon.

Es werden 1H-Pyrido-[2,3-b][1,4]-thiazine der Formel (I) bevorzugt, bei denen

$R^1$ Wasserstoff, Halogen, gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Alkoxy ($C_1$ bis $C_8$) oder die Gruppe

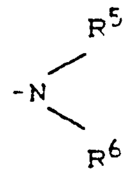

in der

$R^5$ und $R^6$ gleich oder verschieden sind und Wasserstoff, gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Aryl ($C_6$ bis $C_{12}$) bedeuten oder durch eine Kohlenwasserstoffkette zu einem 5- oder 6-gliedrigen Heterocyclus verbunden sein können,

bedeutet,

$R^2$ und $R^3$ gleich oder verschieden sind und Wasserstoff oder gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Alkoxy ($C_1$ bis $C_8$) bedeuten,

$R^4$ Hydroxy oder durch Halogen, Hydroxy, Amino, Dimethylamino, Diethylamino, Cyano, Methoxycarbonyl oder Ethoxycarbonyl substituiertes Alkoxy ($C_1$ bis $C_8$) oder die Gruppe

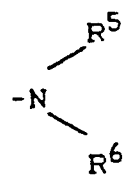

in der

$R^r$ und $R^6$ die obengenannte Bedeutung haben,

oder für den Fall, daß $R^3$ Wasserstoff oder gegebenenfalls substituiertes Alkoxy bedeutet, auch Alkoxy ($C_1$ bis $C_8$)

bedeutet,

wobei

$R^3$ und $R^4$ über eine Polymethylenkette zu einem 5- bis 7-gliedrigen Carbocyclus verbunden sein können

wobei im Falle eines 6-gliedrigen Carbocyclus $R_1$ nicht die Bedeutung Wasserstoff, Chlor, Methoxy oder Ethoxy hat,

und deren quarternären Salze.

Besonders bevorzugt werden 1H-Pyrido-[2,3-b][1,4]-thiazine der Formel (I), bei denen

$R^1$ Wasserstoff, Fluor, Chlor, Brom, gegebenenfalls durch Chlor, Brom, Hydroxy, Amino, Dimethylamino, Diethylamino, Cyano, Methoxycarbonyl oder Ethoxycarbonyl substituiertes Niederalkyl oder Niederalkoxy oder die Gruppe

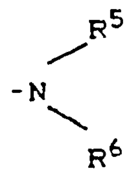

in der

$R^5$ und $R^6$ gleich oder verschieden sind und Wasserstoff, gegebenenfalls durch Chlor, Brom, Hydroxy, Amino, Dimethylamino, Diethylamino, Cyano, Methoxycarbonyl oder Ethoxycarbonyl substituiertes Niederalkyl oder Phenyl bedeuten oder zu einem Morpholin- oder Pyperazinring verbunden sein können,

bedeutet,

$R^2$ und $R^3$ gleich oder verschieden sind und Wasserstoff oder gegebenenfalls durch Chlor, Brom, Hydroxy,

Amino, Dimethylamino, Diethylamino, Cyano, Methoxycarbonyl oder Ethoxycarbonyl substituiertes Niederalkyl oder Niederalkoxy bedeuten,

$R^4$ Hydroxy oder durch Chlor, Brom, Hydroxy, Amino, Dimethylamino, Diethylamino, Cyano, Methoxycarbonyl oder Ethoxycarbonyl substituiertes Niederalkoxy oder die Gruppe

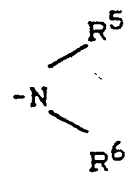

in der

$R^5$ und $R^6$ die obengenannte Bedeutung haben,

oder für den Fall, daß $R^3$ Wasserstoff oder Niederalkoxy bedeutet, auch Niederalkoxy,

bedeutet,

wobei

$R^3$ und $R^4$ über eine Polymethylenkette zu einem 5- bis 7-gliedrigen Carbocyclus verbunden sein können

wobei im Falle eines 6-gliedrigen Carbocyclus $R_1$ nicht die Bedeutung Wasserstoff, Chlor, Methoxy oder Ethoxy hat,

und deren quarternären Salze.

Quaternäre Salze der erfindungsgemäßen 1H-Pyrido-[2,3-b][1,4]-thiazine entstehen durch Umsetzung mit Halogeniden der Formel

$$R^7 - X \qquad (II),$$

in der

$R^7$ Alkyl und

X eine nukleofuge Gruppe bedeutet.

Bevorzugt werden die quaternären Salze durch Umsetzung mit Halogeniden der Formel II

$$R^7 - X$$

in der

$R^7$ Niederalkyl und

X Halogen bedeutet,

hergestellt.

Beispielsweise seien die folgenden 1H-Pyrido-[2,3-b][1,4]-thiazine und deren quaternäre Salze genannt:

| $R^1$ | $R^2$ | $R^3$ | $R^4$ |
|---|---|---|---|
| H | H | H | $OCH_3$ |
| H | H | H | $O-C_2H_5$ |
| H | H | H | $O-CH(CH_3)_2$ |
| H | H | H | $O-CH_2CH_2CH_2CH_3$ |
| H | H | H | $O-CH_2CH(CH_3)_2$ |
| H | H | H | $OC(CH_3)_3$ |
| H | H | H | $O-(CH_2)_4CH_3$ |
| H | H | H | $O-(CH_2)_5CH_3$ |
| H | H | H | $O-CH_2-$ 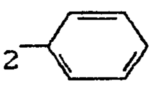 |
| H | H | H | $O-CH_2-CH-$ 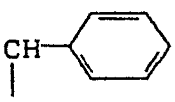 $CH_3$ |
| Cl | H | H | $O(CH_2)_3CH_3$ |
| Cl | H | H | $O-CH_2-$ 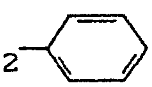 |
| Cl | H | H | $OCH_2CH_2-$ 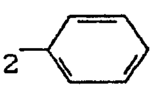 |
| Cl | $CH_2COOC_2H_5$ | H | $O(CH_2)_3CH_3$ |
| $N(CH_3)_2$ | H | H | $O(CH_2)_3CH_3$ |
| $N(C_2H_5)_2$ | H | H | $O-CH_2-$ 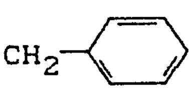 |
| 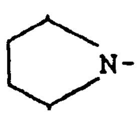 | H | H | $OCH_2CH_2-$ 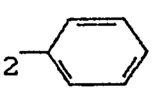 |

| $R^1$ | $R^2$ | $R^3$ | $R^4$ |
|---|---|---|---|
| 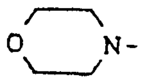 | H | H | $OC_2H_5$ |
| 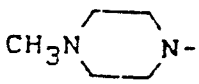 | H | H | $O(CH_2)_3CH_3$ |
| H | H | H | $NH_2$ |
| H | H | H | $NHC_2H_5$ |
| H | H | H | $NHC(CH_3)_3$ |
| H | H | H | NH– 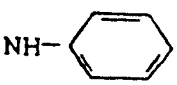 |
| H | H | H | $NH-(CH_2)_2-OH$ |
| H | H | H | $NH-(CH_2)_2-NH_2$ |
| H | H | H | $NH-(CH_2)_3-N(C_2H_5)_2$ |
| H | H | H | $N(C_2H_5)_2$ |
| H | H | H | 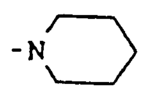 |
| H | H | H | 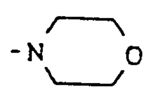 |

| $R^1$ | $R^2$ | $R^3$ | $R^4$ |
|---|---|---|---|
| H | H | H | 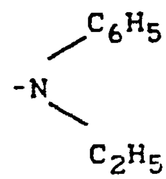 |
| H | H | $CH_3$ | $N(C_2H_5)_2$ |
| H | H | $C_2H_5$ | $NH-(CH_2)_3-N(C_2H_5)_2$ |
| H | H | $C_2H_5$ | 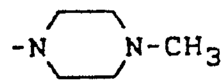 |
| Cl | H | $C_2H_5$ | 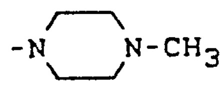 |
| $N(C_2H_5)$ | H | H | 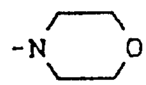 |
| 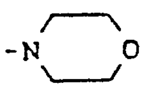 | $CH_2CN$ | $C_2H_5$ | 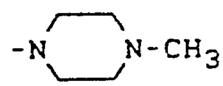 |
| H | H | $R_3$ oder $R_4$ | $-CH_2-CH_2-CH_2-CH_2$ |
| Cl | H | $R_3$ oder $R_4$ | $-CH_2-CH_2-CH_2-CH_2$ |
| H | H | $R_3$ oder $R_4$ | $-CH_2-CH_2$ |
| Cl | H | $R_3$ oder $R_4$ | $-CH_2-CH_2$ |
| $N(C_2H_5)_2$ | H | $R_3$ oder $R_4$ | $-CH_2-CH_2$ |

Salze:

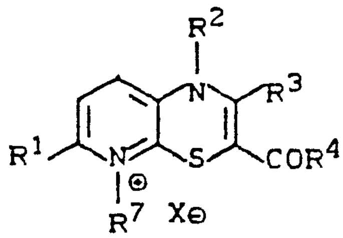

(Ia)

| $R_1$ | $R_2$ | $R_3$ | $R_4$ | $R_7$ | X |
|---|---|---|---|---|---|
| H | H | H | $OC_2H_5$ | $CH_3$ | I |
| H | H | H | $OC_2H_5$ | $C_2H_5$ | I |
| H | H | H | $OC_2H_5$ | $CH_2COOC_2H_5$ | Br |
| H | H | H | $O(CH_2)_3CH_3$ | $C_2H_5$ | Cl |
| Cl | H | H | $N(C_2H_5)_2$ | $C_2H_5$ | I |

Es wurde auch ein Verfahren zur Herstellung von 1H-Pyrido-[2,3-b][1,4]-thiazinen gefunden, das dadurch gekennzeichnet ist, daß man Pyridinverbindungen der Formel

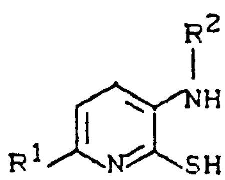

(III),

in der

$R^1$ Wasserstoff, Halogen, gegebenenfalls substituiertes Alkyl oder Alkoxy oder die Gruppe

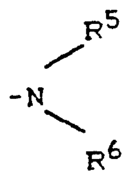

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet, und

$R^2$ Wasserstoff oder gegebenenfalls substituiertes Alkyl oder Alkoxy bedeutet,

mit Carbonylverbindungen der Formel

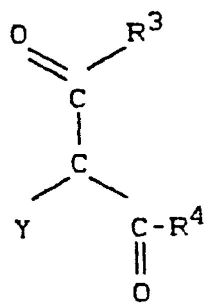

(IV),

in der

Y für Halogen steht,

$R^3$ Wasserstoff oder gegebenenfalls substituiertes Alkyl oder Alkoxy bedeuten und

$R^4$ Hydroxy, gegebenenfalls substituiertes Alkoxy oder die Gruppe

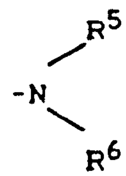

in der

$R^5$ und $R^6$ die obengenannte Bedeutung haben

bedeutet,

wobei

$R^3$ und $R^4$ über eine Polymethylenkette zu einem 5- bis 7-gliedrigen Carbocyclus verbunden sein können,

in Gegenwart einer Base gegebenenfalls in Gegenwart von Lösungsmitteln, umsetzt.

Das erfindungsgemäße Verfahren kann beispielsweise durch das folgende Reaktionsschema dargestellt werden:

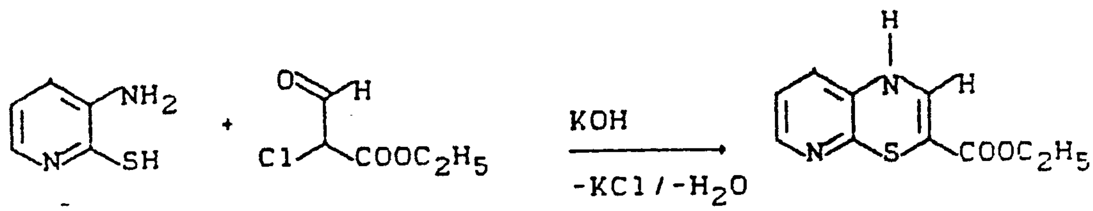


Die Pyridin-Verbindungen für das erfindungsgemäße Verfahren sind an sich bekannt und lassen sich nach bekannten Verfahren herstellen (Pyridine and its Derivatives, Part IV, 345-437, in The Chemistry of Heterocyclic Compounds, Editor: E. Klingsberg, John Wiley, New York - London - Sydney, 1964; C.O. Okafor, Int. J. Sulfur Chem. B, 7, 121-153 (1972) sowie C.O. Okafor, J. Org. Chem. 38, 4383 (1973)).

Die Carbonylverbindungen für das erfindungsgemäße Verfahren sind ebenfalls bekannt und können nach bekannten Methoden hergestellt werden (K.H. Dudley et al., J. Heterocyclic Chem. 10, 938 (1973); Org. Synth. 21, 4, (1941) sowie C. Bülow u. E. King, Liebigs Ann. Chem. 439, 211 (1924)).

Nukleofuge Gruppen können beispielsweise Halogen (Fluor, Chlor Brom oder Iod) und O-Tosyl sein.

Da bei der Reaktion Halogenwasserstoff frei wird, ist es zweckmäßig, in Gegenwart von Basen zu arbeiten. Dafür kommen anorganische Basen, wie Alkali- oder Erdalkalihydroxide oder -carbonate bzw. die entsprechenden Ammoniumverbindungen, sowie organische Basen wie Triethylamin oder Pyridin in Frage.

Als Verdünnungsmittel können alle inerten organischen Lösungsmittel eingesetzt werden. Hierzu gehören vorzugsweise Ether wie Dioxan und Tetrahydrofuran, Alkohole wie Methanol, Ethanol oder Isopropanol, dipolare aprotische Solventien wie Dimethylformamid, Dimethylsulfoxid oder N-Methylpyrrolidon sowie Wasser.

Das erfindungsgemäße Verfahren kann durchgeführt werden in Gegenwart von ausschließlich einem oder mehreren organischen Lösungsmitteln oder Wasser und einem oder mehreren mit Wasser nicht mischbaren Lösungsmitteln.

Die Reaktionstemperaturen können in einem größeren Bereich variiert werden. Im allgemeinen arbeitet man zwischen etwa $-10^{0}$ C und etwa $+100^{0}$ C, vorzugsweise zwischen $0^{0}$ C und $+60^{0}$ C. Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens kann es zweckmäßig sein, in Inertgasatmosphäre zu arbeiten, bevorzugt unter Stickstoff. Zur Darstellung der erfindungsgemäßen Verbindungen setzt man bevorzugt Pyridinverbindungen, Carbonylverbindungen sowie Basen im Molverhältnis 1:1:1 um.

Die Aufarbeitung der Reaktionsansätze zur Isolierung der erfindungsgemäßen Verbindungen erfolgt durchweg in allgemein bekannter Art und Weise.

Im Rahmen der vorliegenden Erfindung seien als besondere Ausführungsformen die Varianten A und B genannt:

Variante A
Nach diesem Verfahren können bevorzugt erfindungsgemäß Säureamide der 1H-Pyrido-[2,3-b][1,4]-thiazine hergestellt werden, indem man Ester der 1H-Pyrido-[2,3-b][1,4]-thiazine der Formel

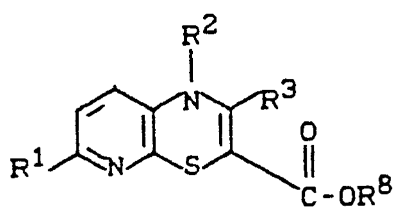

(V),

in der

$R^1$ Wasserstoff, Halogen, gegebenenfalls substituiertes Alkyl oder Alkoxy oder die Gruppe

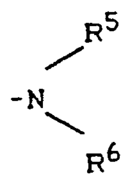

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^8$ für Alkyl steht,

mit Aminen der Formel

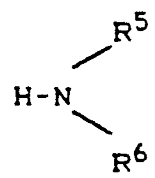

(VI),

in der

$R^5$ und $R^6$ die obengenannte Bedeutung haben,

umsetzt.

Verwendet man beispielsweise 3-Methoxycarbonyl-1H-pyrido-[2,3-b][1,4]-thiazin und Aminoethanol als Ausgangsstoffe, so kann der Reaktionsablauf durch das folgende Formelschema wiedergegeben werden:

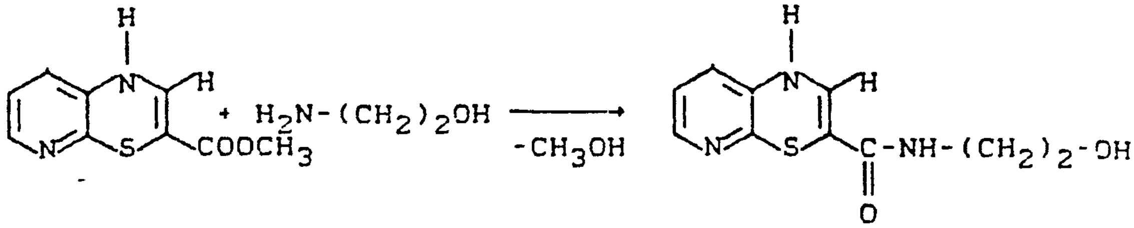

Die erfindungsgemäß verwendbaren Ester der Formel (V) können erfindungsgemäß wie oben angegeben durch Umsetzung der Pyridinverbindungen der Formel (III) mit Carbonylverbindungen der Formel (IV) hergestellt werden.

Die Amine für das erfindungsgemäße Verfahren sind bekannt (Houben Weyl, Bd. 11/1, S. 9 bis 1026, 1957). Als Verdünnungsmittel kommen alle inerten organischen Lösungsmittel in Frage.

Hierzu gehören vorzugsweise chlorierte Kohlenwasserstoffe, wie Methylenchlorid, Chloroform, Tetrachlorkohlenstoff, 1,2-Dichlorethan, 1,2,2-Trichlorethan und Chlorbenzol, Alkohole, vorzugsweise Methanol, Ethanol sowie Isopropanol, Ether wie Dioxan oder Tetrahydrofuran sowie dipolar aprotische Solventien wie Dimethylformamid, Dimethylsulfoxid oder N-Methylpyrrolidon.

Das erfindungsgemäße Verfahren kann durchgeführt werden in Gegenwart von ausschließlich einem oder mehreren organischen Lösungsmitteln.

Besonders bevorzugt ist eine Verfahrensweise, bei der das erfindungsgemäße Amin, falls es flüssig ist, nicht nur als Reagenz, sondern auch als Solvens eingesetzt wird.

Die Reaktionstemperaturen können in einem größeren Bereich variiert werden. Im allgemeinen arbeitet man zwischen etwa 0° C und etwa 100° C, vorzugsweise zwischen 0° C und +60° C. Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens kann man die Ausgangsstoffe in äquimolaren Mengen einsetzen, vorteilhaft ist jedoch die Verwendung eines Überschusses an Amin.

Die Aufarbeitung der Reaktionsansätze zur Isolierung der erfindungsgemäßen Verbindungen erfolgt durchweg in allgemein bekannter Art und Weise.

Variante B

Nach diesem Verfahren können bevorzugt in 1-Stellung substituierte 1H-Pyrido-[2,3-b][1,4]-thiazine hergestellt werden, indem man 1H-Pyrido-[2,3-b][1,4]thiazine der Formel

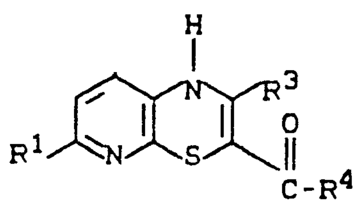

(VII),

in der

$R^1$ Wasserstoff, Halogen, gegebenenfalls substituiertes Alkyl oder Alkoxy oder die Gruppe

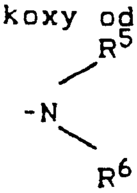

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^3$ Wasserstoff oder gegebenenfalls substituiertes Alkyl oder Alkoxy bedeuten und

$R^4$ Hydroxy, gegebenenfalls substituiertes Alkoxy oder die Gruppe

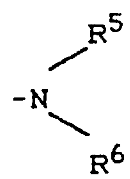

in der

$R^5$ und $R^6$ die obengenannte Bedeutung haben,

bedeutet,

wobei

$R^3$ und $R^4$ über eine Polymethylenkette zu einem 5- bis 7-gliedrigen Carbocyclus verbunden sein können,

mit Halogeniden oder Tosylaten der Formel

$$R^2 - Z \qquad (VIII),$$

in der

Z Halogen oder O-Tosyl bedeutet und

$R^2$ gegebenenfalls substituiertes Alkyl oder Alkoxy bedeutet,

in Gegenwart von Basen umsetzt.

Verwendet man beispielsweise 3-Ethoxycarbonyl-1H-pyrido-[2,3-b][1,4]-thiazin und Bromessigsäureethylester als Ausgangsmaterialien, so läßt sich der Reaktionsablauf durch das folgende Formelschema wiedergeben:

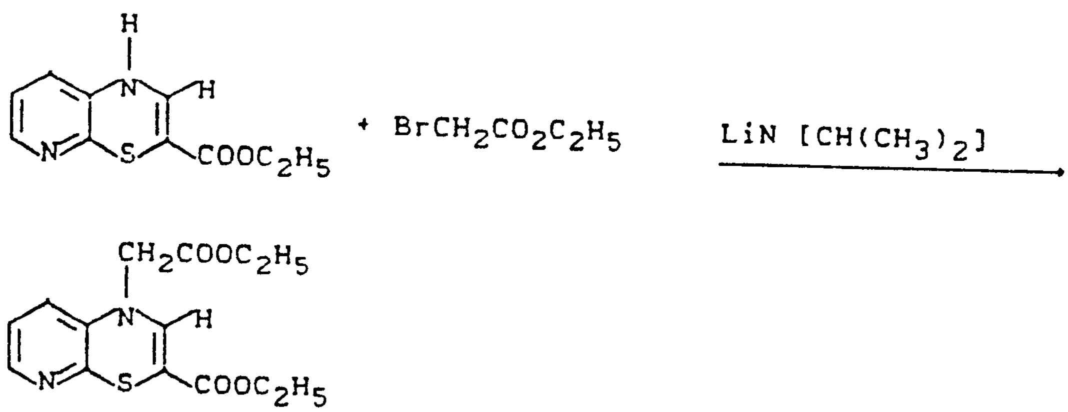


Die in 1-Stellung unsubstituierten 1H-Pyrido-[2,3-b]-[1,4]-thiazine können erfindungsgemäß wie oben angegeben durch Umsetzung der Pyridinverbindungen der Formel (III) mit Carbonylverbindungen der Formel (IV) hergestellt werden.

Die Halogenide oder Tosylate sind bekannt (S.R. Sandler u. W. Karo, Organic Functional Group Preparations Bd. I, S. 148-179, Academic Press (1983)).

Als Verdünnungsmittel kommen inerte organische Lösungsmittel in Frage.

Hierzu gehören vorzugsweise Ether wie Dioxan, Tetrahydrofuran sowie Mono- oder Diglyme und dipolar aprotische Solventien wie Dimethylformamid, Hexamethylphosphorsäuretriamid oder N-Methylpyrrolidon.

Das erfindungsgemäße Verfahren kann durchgeführt werden in Gegenwart von ausschließlich einem oder mehreren organischen Lösungsmitteln.

Als erfindungsgemäß verwendbare Basen kommen beispielsweise Natriumhydrid, metallorganische Reagenzien wie Butyllithium, Phenyllithium, Lithiumdiisopropylamid Grignard-Verbindungen wie Methylmagnesiumiodid sowie Alkalialkoholate wie Kalium-tert.-butylat in Betracht.

Die Reaktionstemperaturen können in einem größeren Bereich variiert werden. Im allgemeinen arbeitet man zwischen etwa -100° C und etwa +100° C, vorzugsweise zwischen -70° C und +50° C. Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens kann man die Ausgangsstoffe in äquimolaren Mengen einsetzen, vorteilhaft kann die Verwendung eines Überschusses an Halogenid oder Tosylat und der Base sein.

Die Aufarbeitung der Reaktionsansätze zur Isolierung der erfindungsgemäßen Verbindungen erfolgt durchweg in allgemein bekannter Art und Weise.

Die Herstellung der quaternären Salze der 1H-Pyrido-[2,3-b][1,4]-thiazine kann beispielsweise durch das folgende Formelschema wiedergegeben werden:

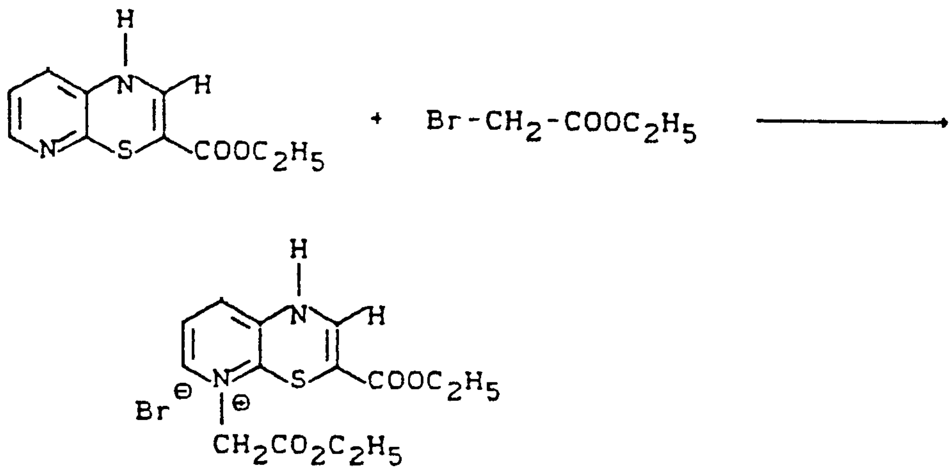

Bevorzugt führt man die Quaternierung mit Alkylhalogeniden oder Tosylaten durch. Die Alkylhalogenide oder Tosylate sind bekannt (S.R. Sandler u. W. Karo, Organic Functional Group Preparations, Bd. I, S. 148-179, Academic Press (1983)).

Als Verdünnungsmittel kommen alle inerten organischen Lösungsmittel in Frage. Hierzu gehören vorzugsweise Lösungsmittel mit hohem Dipolmoment wie Nitromethan, Nitrobenzol oder Acetonitril. Weitere, ebenfalls geeignete Solventien findet man in Houben-Weyl, Bd. 11/2, S. 596.

Das erfindungsgemäße Verfahren kann in Gegenwart von ausschließlich einem oder mehreren Lösungsmitteln ausgeführt werden.

Die Reaktionstemperaturen können in einem größeren Bereich variiert werden. Im allgemeinen arbeitet man zwischen etwa 0° C und etwa +200° C, vorzugsweise zwischen +20° C und +200° C. Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens kann man die Reaktionspartner im äquimolaren Verhältnis einsetzen, es kann aber zweckmäßig sein, eine Komponente im Überschuß anzuwenden.

Die Aufarbeitung der Reaktionsansätze zur Isolierung der erfindungsgemäßen Verbindungen erfolgt durchweg in allgemein bekannter Art und Weise. Der Austausch der Anionen erfolgt ebenfalls in allgemein bekannter Art und Weise.

Die quaternären Salze können durch die Formel

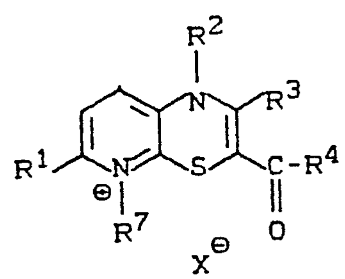

in der

$R^1$ bis $R^4$, $R^7$ und X die obengenannte Bedeutung haben,

hergestellt werden.

Die erfindungsgemäßen 1H-Pyrido-[2,3-b][1,4]-thiazine sind Wirkstoffe für Arzneimittel und zur therapeutischen Behandlung von Menschen und Tieren geeignet. Sie sind Lipoxygenasehemmer und besonders zur Behandlung von entzündlichen, allergischen und asthmatischen Erkrankungen geeignet.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Beispiel 1

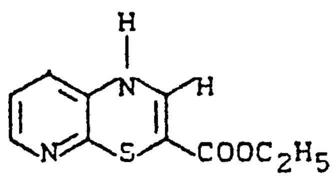

3-Ethoxycarbonyl-1H-pyrido[2,3-b][1,4]-thiazin

Unter Stickstoff gibt man bei 25° C zu einer Lösung von 4 g (0,0317 Mol) 2-Mercapto-3-aminopyridin und 1,78 g (0,0317 Mol) Kaliumhydroxid in 200 ml Ethanol 4,8 g α-Chlor-formylessigsäureethylester, gelöst in 70 ml Ethanol. Man erwärmt 1 Stunde auf 50° C, zieht anschließend das Ethanol im Vakuum ab und chromatographiert den Rückstand an Kieselgel mit Dichlormethan/Methanol (95:5) als Laufmittel.

Ausbeute: 3,3 g (47 % der Theorie)
Fp.: 194-195° C

Analog wurden hergestellt:

| Beispiel Nr. | Formel | Schmp. °C | Ausbeute % der Theorie |
|---|---|---|---|
| 2 | 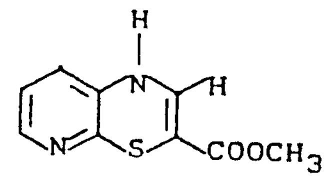 | 199-201 | 86 |
| 3 | 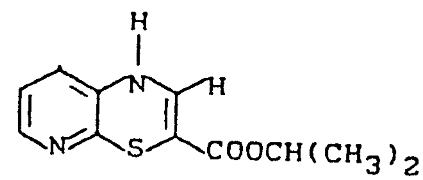 | 172-174 | 34 |
| 4 | 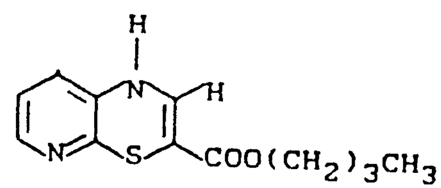 | 156-158 | 45 |

| Beispiel Nr. | Formel | Schmp. °C | Ausbeute % der Theorie |
|---|---|---|---|
| 5 | 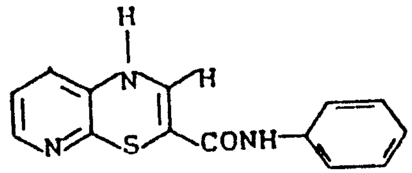 | 193-198 | 68 |
| 6 | 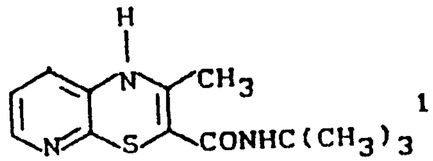 | 118-121 | 52 |
| 7 | 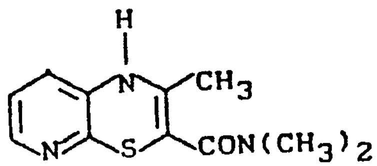 | | |
| 8 | 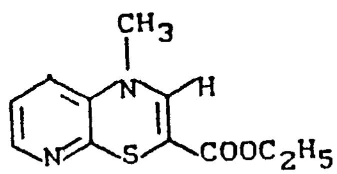 | 113-115 | 63 |
| 9 | 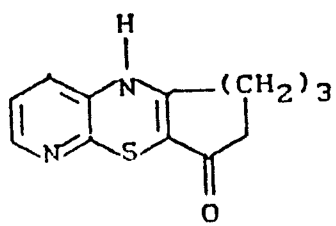 | 265-268 | 71 |

0226023

Beispiel 10

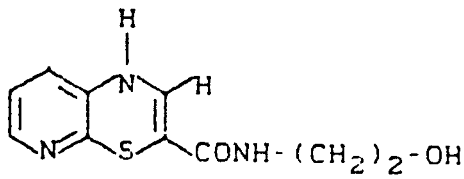

3-[(2-Hydroxyethylamino)carbonyl]-1H-pyrido[2,3-b][1,4]-thiazin

---

0,52 g (0,0025 Mol) 3-Methoxycarbonyl-1H-pyrido[2,3-b]-[1,4]-thiazin werden bei 25° C 25 Stunden mit 5 ml Aminoethanol behandelt. Danach wird das überschüssige Reagenz im Vakuum abdestilliert und der Rückstand an Kieselgel chromatographiert, Laufmittel: Dichlormethan/Methanol (86:14).

Ausbeute: 0,5 g (84 % der Theorie)
Fp.: 192-195° C

Analog wurden hergestellt:

| Beispiel Nr. | Formel | Schmp. °C | Ausbeute % |
| --- | --- | --- | --- |
| 11 | 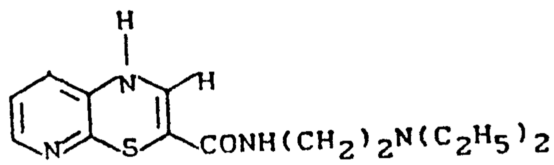 | 181-186 | 82 |
| 12 | 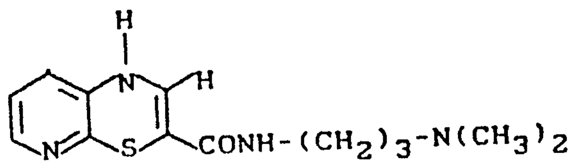 | >160 (Zers.) | 92 |
| 13 | 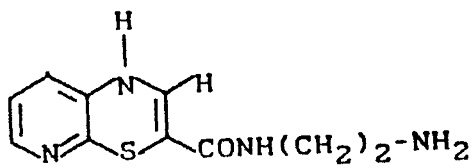 | 130-137 | 50 |

0226023

Beispiel 14

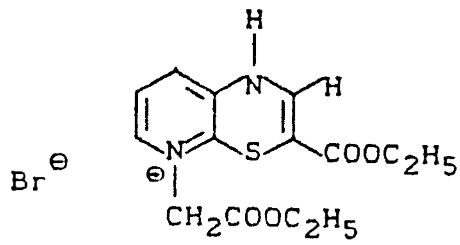

Zu einer Lösung von 0,5 g (0,00225 Mol) 3-Ethoxycarbonyl-1H-pyrido[2,3-b][1,4]-thiazin in 10 ml Nitromethan gibt man 3,31 g (0,0185 Mol) Bromessigsäureethylester und erhitzt das Reaktionsgemisch 1 Stunde am Rückfluß. Anschließend kühlt man auf 25° C ab und versetzt mit Diethylether bis zur beginnenden Kristallisation. Die beim Stehenlassen ausgefallenen Kristalle werden abgesaugt und getrocknet.

Ausbeute: 0,35 g (40 % der Theorie)
Fp.: 158-159° C

Analog wurden hergestellt:

| Beispiel Nr. | Formel | Fp. °C | Ausbeute % der Theorie |
|---|---|---|---|
| 15 | 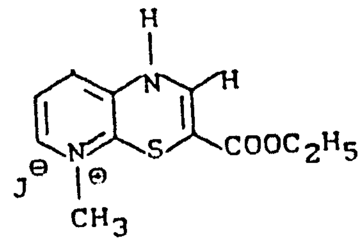 | 195-198 | 37 |
| 16 | 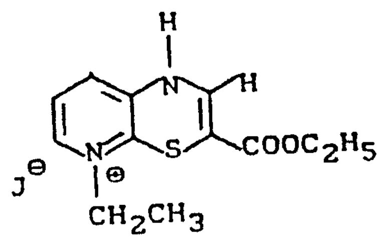 | 142-152 | 49 |

0226023

Beispiel 17

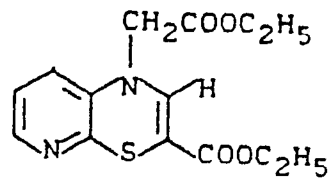

Zu einer auf -20°C gekühlten Lösung von 0,36 g (0,00351 Mol) Diisopropylamin in 10 ml THF gibt man unter Rühren, Stickstoff und Feuchtigkeitsausschluß 2,02 ml einer 1,6 mol. Lösung von n-Butyllithium in n-Hexan (0,00321 Mol). Man läßt 10 min rühren, kühlt auf -70°C ab und tropft eine Lösung von 0,5 g (0,00225 Mol) 3-Ethoxycarbonyl-1H-pyrido[2,3-b][1,4]-thiazin in 10 ml THF zu. Nach Zusatz von 0,5 g Hexamethylphosphorsäuretriamid läßt man die Temperatur der Reaktionsmischung wieder auf -20°C ansteigen und tropft eine Lösung von 0,63 g (0,00351 Mol) Bromessigsäureethylester in 5 ml THF zu. Man läßt bis zum vollständigen Umsatz bei 25°C rühren. Danach verdünnt man mit 200 ml Dichlormethan, schüttelt mit 4 mal 50 ml Wasser aus, trocknet die organische Phase über wasserfreiem Natriumsulfat und engt ein. Der Rückstand wird an Kieselgel chromatographiert, Laufmittel Dichlormethan/Methanol (97:3).

Ausbeute: 0,54 g (78 % der Theorie)

Fp.: 152-155°C

Analog wurden hergestellt:

| Beispiel Nr. | Formel | Schmp. °C | Ausbeute % der Theorie |
| --- | --- | --- | --- |
| 18 | 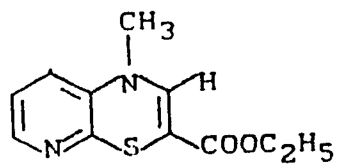 | 112-115 | 67 |
| 19 | 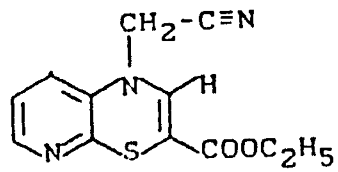 | 157-158 | 72 |

## Anwendungsbeispiele

### Beispiel 20

Der Nachweis der lipoxygenasehemmenden Eigenschaften der 1H-Pyrido[2,3-b][1,4]-thiazine erfolgt analog den Methoden von

Borgeat, P. Samuelsson, B. (1979)
Proc. Nat. Acad. Sci. 76, 2148-2152 und
Hamilton, J.G., Karol, R.J. (1982),
Prog. Lipid Res. 21, 155-170

Polymorphkernige Rattenleukozyten (PMNL) wurden aus dem Peritonealraum von Wistar-Ratten, 18 Stunden nach i.p.-Applikation von 6 ml einer 12 %igen Natriumcaseinat-Suspension gewonnen.

Als Maß für eine Lipoxygenase-Hemmung wurde die Freisetzung von $LTB_4$ an polymorphkernigen Granulozyten nach Zugabe von Substanzen und Calcium-Ionophor mittels HPLC bestimmt.

Nach Zentrifugation und Waschen der PMNL mit Inkubationspuffer (137 mN NaCl; 2,7 mM KCl; 5,0 mM $Na_2HPO_4$; 5,55 mM Glucose ; 2,0 mM $CaCl_2$, pH = 7,2) wurde die Zelldichte auf 2 x $10^7$ ml eingestellt (Coulter Counter) und 1 ml dieser Zellsuspension mit 2,5 σl DMSO bzw. 2,5 σl verschiedener Testsubstanzkonzentrationen in DMSO über 5 min bei 37°C vorinkubiert. Nach Stimulation der Zellen mit 2,5 σl Calcium-Ionophor A 23187 (1 mg/ml DMSO) wurde die 6-minütige Hauptinkubation durch Zugabe von 1,5 ml

0226023

$PGB_2$-haltigem Methanol gestoppt (1 µl/ml), 2 ml zellfreier Überstand durch Zentrifugation (1000 g, 3 min, RT) gewonnen und nach Ansäuern mit 1 N HCl auf pH 3,0 zweimal mit 4 ml Ether extrahiert. Die vereinigten Etherphasen wurden mit 4 ml Wasser (Bidest) gewaschen, unter Stickstoff-Begasung getrocknet und in 80 µl Methanol aufgenommen.

Von jeder der so aufbereiteten Proben wurden 20 µl auf eine Fertigsäle (Nucleosil, Typ 7,5 C 18M 4 x 25 mm) aufgetragen und bei einer Durchflußrate von 1 ml/min (Kontron Pumpensystem 600) chromatographiert, wobei als mobile Phase Methanol, $H_2O$ und Essigsäure (75:25:0:0,01) diente. Die Detektion erfolgte bei 280 nm (Uvicon 720 LC). Die Bildung des Metaboliten wurde mit Hilfe des internen Standards PG $B_2$ als Quotient der Flächenintegrale (Shimadzu C-R1B) von $LTB_4$ zu $PGB_2$ quantifiziert und eine Hemmung als Prozent der Kontrollen ermittelt. Wie aus der nachfolgenden Tabelle ersichtlich, bewirken die 1H-Pyrido[2,3-b][1,4]-thiazine eine signifikante Hemmung der $LTB_4$-Biosynthese in Rattengranulozyten.

Hemmung der Leukotrien $B_4$ - Biosynthese ($IC_{50}$)

| Verbindung aus Beispiel Nr. | M/l |
|---|---|
| 1 | $4,6 \cdot 10^{-7}$ |
| 2 | $5 \cdot 10^{-6}$ |
| 4 | $5,5 \cdot 10^{-8}$ |
| 19 | $2,6 \cdot 10^{-6}$ |
| 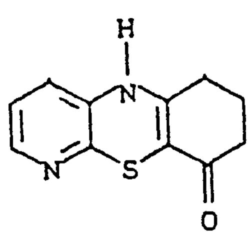 | 42 % bei $1 \cdot 10^{-5}$ |

0226023

Die erfindungsgemäßen 1H-Pyrido[2,3-b][1,4]-thiazine sind auch in vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66, 81 (1980)).

Beispielhaft sei die Wirkung eines 1H-Pyrido-[2,3-b][1,4]-thiazins nach lokaler Applikation, durch Einführen eines mit Wirkstoff getränkten Schwämmchens unter die Rückenhaut von Ratten, erwähnt.

| Verbindung Nr. | Dosis,lokal (mg/Ratte) | Hemmung der Leukozytenmigration (Kontrolle = 0 %) |
|---|---|---|
| 1 | 10 | 50 |

Beispiel 21

Die antiasthmatische Wirkung der erfindungsgemäßen Verbindung kann ebenfalls nach bereits bekannten Methoden nachgewiesen werden (vgl. Samuelson et al., FEBS Letters, 110, 213 (1980) und Yen et al., Agents and Actions 10, 274 (1980)).

## Patentansprüche

1. 1H-Pyrido-[2,3-b][1,4]-thiazine der Formel

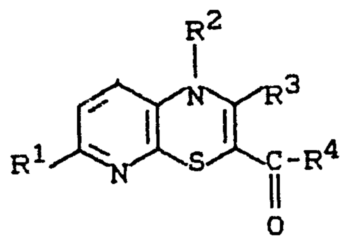

(I),

in der

$R^1$ Wasserstoff, Halogen, gegebenenfalls substituiertes Alkyl oder Alkoxy oder die Gruppe

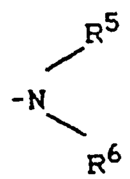

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy oder die Gruppe

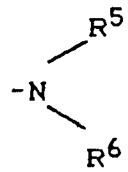

in der

$R^5$ und $R^6$ die obengenannte Bedeutung haben,

oder für den Fall, daß $R^3$ Wasserstoff oder gegebenenfalls substituiertes Alkoxy bedeutet, auch gegebenenfalls substituiertes Alkoxy

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

und deren quaternäre Salze.

2. 1H-Pyrido-[2,3-b][1,4]-thiazine nach Anspruch 1, wobei

$R^1$ Wasserstoff, Halogen, gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Alkoxy ($C_1$ bis $C_8$) oder die Gruppe

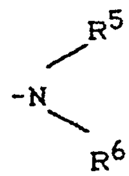

in der

$R^5$ und $R^6$ gleich oder verschieden sind und Wasserstoff, gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Aryl ($C_6$ bis $C_{12}$) bedeuten oder zu einem 5-oder 6-gliedrigen Heterocyclus verbunden sein können,

bedeutet,

$R^2$ und $R^3$ gleich oder verschieden sind und Wasserstoff oder gegebenenfalls durch Halogen, Hydroxy, Amino, Dimethylamino, Diethylamino, Cyano, Methoxycarbonyl oder Ethoxycarbonyl substituiertes Alkyl ($C_1$ bis $C_8$) oder Alkoxy ($C_1$ bis $C_8$) bedeuten,

$R^4$ Hydroxy oder durch Halogen, Hydroxy, Amino, Dimethylamino, Diethylamino, Cyano, Methoxycarbonyl oder Ethoxycarbonyl substituiertes Alkoxy ($C_1$ bis $C_8$) oder die Gruppe

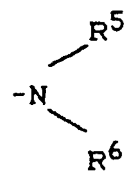

in der

$R^5$ und $R^6$ die obengenannte Bedeutung haben,

oder für den Fall, daß $R^3$ Wasserstoff oder gegebenenfalls substituiertes Alkoxy bedeutet, auch Alkoxy ($C_1$ bis $C_8$)

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

und deren quarternären Salze.

3. 1H-Pyrido-[2,3-b][1,4]-thiazine nach den Ansprüchen 1 und 2, wobei

$R^1$ Wasserstoff, Fluor, Chlor, Brom, gegebenenfalls durch Chlor, Brom, Hydroxy, Amino, Dimethylamino, Diethylamino, Cyano, Methoxycarbonyl oder Ethoxycarbonyl substituiertes Niederalkyl oder Niederalkoxy oder die Gruppe

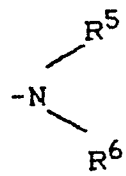

in der

$R^5$ und $R^6$ gleich oder verschieden sind und Wasserstoff, gegebenenfalls durch Chlor, Brom, Hydroxy, Amino, Dimethylamino, Diethylamino, Cyano, Methoxycarbonyl oder Ethoxycarbonyl substituiertes Niederalkyl oder Phenyl bedeuten oder zu einem Morpholin- oder Piperazinring verbunden sein können,

bedeutet,

$R^2$ und $R^3$ gleich oder verschieden sind und Wasserstoff oder gegebenenfalls durch Chlor, Brom, Hydroxy, Amino, Dimethylamino, Diethylamino, Cyano, Methoxycarbonyl oder Ethoxycarbonyl substituiertes Niederalkyl oder Niederalkoxy bedeuten,

$R^4$ Hydroxy oder gegebenenfalls durch Chlor, Brom, Hydroxy, Amino, Dimethylamino, Diethylamino, Cyano, Methoxycarbonyl oder Ethoxycarbonyl substituiertes Niederalkoxy oder die Gruppe

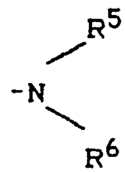

in der

$R^5$ und $R^6$ die obengenannte Bedeutung haben,

oder für den Fall, daß $R^3$ Wasserstoff oder Niederalkoxy bedeutet, auch Niederalkoxy,

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

und deren quarternären Salze.

4. Verfahren zur Herstellung von 1H-Pyrido-[2,3-b][1,4]-thiazinen der Formel

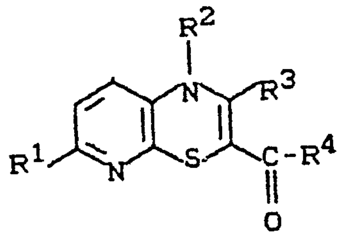

(I),

in der

$R^1$ Wasserstoff, Halogen, gegebenenfalls substituiertes Alkyl oder Alkoxy oder die Gruppe

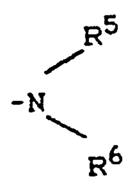

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy oder die Gruppe

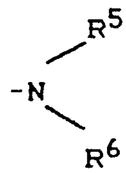

in der

$R^5$ und $R^6$ die obengenannte Bedeutung haben,

oder für den Fall, daß $R^3$ Wasserstoff oder gegebenenfalls substituiertes Alkoxy bedeutet, auch gegebenenfalls substituiertes Alkoxy

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

0226023

dadurch gekennzeichnet, daß man Pyridinverbindungen der Formel

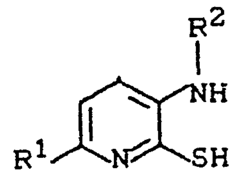

(III),

in der

$R^1$ und $R^2$ die oben genannte Bedeutung haben,

mit Carbonylverbindungen der Formel

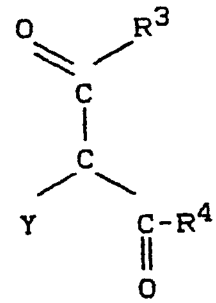

(IV),

in der

Y für Halogen steht, und

$R^3$ und $R^4$ die oben genannte Bedeutung haben,

in Gegenwart einer Base gegebenenfalls in Gegenwart von Lösungsmitteln umsetzt.

5. Verfahren zur Herstellung von Säureamiden der 1H-Pyrido-[2,3-b][1,4]-thiazine, dadurch gekennzeichnet, daß man Ester der 1H-Pyrido-[2,3-b]-[1,4]-thiazine der Formel

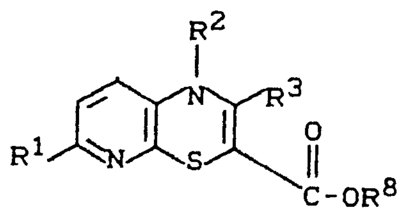

(V),

in der

$R^1$ Wasserstoff, Halogen, gegebenenfalls substituiertes Alkyl oder Alkoxy oder die Gruppe

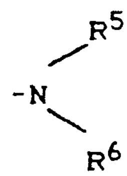

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^8$ für Alkyl steht,

mit Aminen der Formel

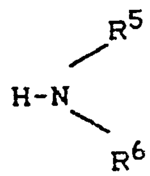

(VI),

in der

$R^5$ und $R^6$ die obengenannte Bedeutung haben,

umsetzt.

6. Verfahren zur Herstellung von in 1-Stellung substituierten 1H-Pyrido-[2,3-b][1,4]-thiazinen, dadurch gekennzeichnet, daß man 1H-Pyrido-[2,3-b][1,4]-thiazine der Formel

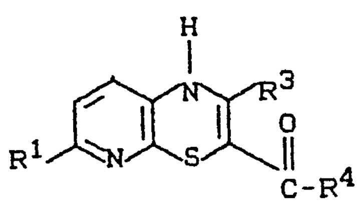

(VII),

in der

$R^1$ Wasserstoff, Halogen, gegebenenfalls substituiertes Alkyl oder Alkoxy oder die Gruppe

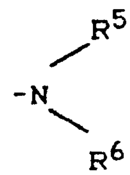

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^3$ Wasserstoff oder gegebenenfalls substituiertes Alkyl oder Alkoxy bedeuten und

$R^4$ Hydroxy, gegebenenfalls substituiertes Alkoxy oder die Gruppe

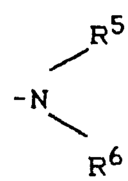

in der

$R^5$ und $R^6$ die obengenannte Bedeutung haben,

0226023

bedeutet,

wobei

$R^3$ und $R^4$ über eine Polymethylenkette zu einem 5- bis 7-gliedrigen Carbocyclus verbunden sein können,

mit Halogeniden oder Tosylaten der Formel

$R^2$ - Z (VIII),

in der

Z Halogen oder O-Tosyl bedeutet und

$R^7$ gegebenenfalls substituiertes Alkyl oder Alkoxy bedeutet,

in Gegenwart von Basen umsetzt.

7. 1H-Pyrido-[2,3-b][1,4]-thiazine der Formel

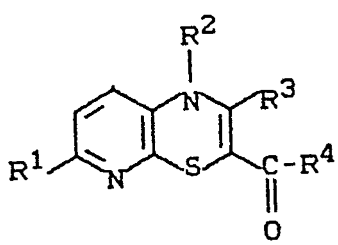

(I),

in der

$R^1$ Wasserstoff, Halogen, gegebenenfalls substituiertes Alkyl oder Alkoxy oder die Gruppe

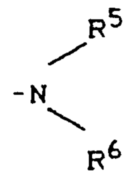

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy oder die Gruppe

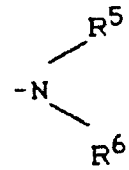

in der

$R^5$ und $R^6$ die obengenannte Bedeutung haben,

oder für den Fall, daß $R^3$ Wasserstoff oder gegebenenfalls substituiertes Alkoxy bedeutet, auch gegebenenfalls substituiertes Alkoxy bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Waserstoff, Chlor, Methoxy oder Ethoxy hat,

und deren quaternären Salze,

zur therapeutischen Behandlung.

8. Arzneimittel enthaltend 1H-Pyrido-[2,3-b][1,4]-thiazine der Formel

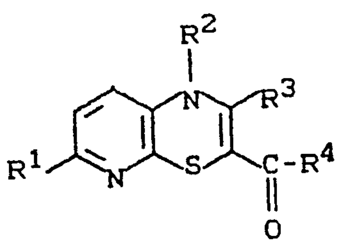

(I),

in der

$R^1$ Wasserstoff, Halogen, gegebenenfalls substituiertes Alkyl oder Alkoxy oder die Gruppe

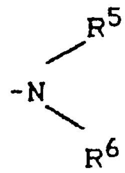

in der

$R^5$ und $R^6$ gleich oder verschieden sind und Wasserstoff, gegebenenfalls substituiertes Alkyl oder Aryl bedeuten oder zu einem 5- oder 6-gliedrigen Heterocyclus verbunden sein können,

bedeutet,

$R^2$ und $R^3$ gleich oder verschieden sind und Wasserstoff oder gegebenenfalls substituiertes Alkyl oder Alkoxy bedeuten und

$R^4$ Hydroxy oder die Gruppe

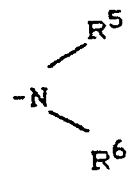

in der

$R^5$ und $R^6$ die obengenannte Bedeutung haben,

oder für den Fall, daß $R^3$ Wasserstoff oder gegebenenfalls substituiertes Alkoxy bedeutet, auch gegebenenfalls substituiertes Alkoxy,

bedeutet,

wobei

$R^3$ und $R^4$ über eine Polymethylenkette zu einem 5- bis 7-gliedrigen Carbocyclus verbunden sein können, wobei im Falle eines 6-gliedrigen Carbocyclus $R^1$ nicht die Bedeutung Wasserstoff, Chlor, Methoxy oder Ethoxy hat,

und deren quaternären Salze.

9. Arzneimittel nach Anspruch 8, dadurch gekennzeichnet, daß es 0,5 bis 90 Gew.-% des Wirkstoffs enthält.

10. Verwendung von 1H-Pyrido-[2,3-b][1,4]-thiazinen nach Anspruch 1 zur Herstellung von Arzneimitteln.

Europäisches Patentamt

# EUROPÄISCHER RECHERCHENBERICHT

Nummer der Anmeldung 0226023

EP 86 11 5312

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | BEILSTENS HANDBUCH DER ORGANISCHEN CHEMIE, Band 27, Teil 11, 3. und 4. Ergänzungswerk, 4. Auflage, Springer-Verlag, 1984, Berlin, DE * Seite 7916, Verbindung der Formel VII; Seite 7956, Verbindung der Formel VI * --- | 1,4 | C 07 D 513/04<br>A 61 K 31/54 //<br>(C 07 D 513/04<br>C 07 D 279:00<br>C 07 D 221:00 ) |
| A | EP-A-0 100 527 (BAYER AG) * Ansprüche 1, 3-6 * --- | 1,8,10 | |
| A,D | CHEMICAL ABSTRACTS, Band 83, Nr. 23, 8. Dezember 1975, Seite 476, Spalte 2, Zusammenfassungs Nr. 193384y; & SU - A - 430 642 (ORDZHONIKIDZE, S., ALL-UNION SCIENTIFIC-RESEARCH CHEMICAL-PHARMACEUTICAL INSTITUTE) 25.07.1975 --- | 1,4 | RECHERCHIERTE SACHGEBIETE (Int Cl 4)<br>C 07 D 513/00<br>A 61 K 31/00 |
| A | EP-A-0 101 898 (BAYER AG) * Ansprüche 1, 3-7 * ----- | 1,8,10 | |

Der vorliegende Recherchenbericht wurde für alle Patentansprüche erstellt

| Recherchenort | Abschlußdatum der Recherche | Prüfer |
|---|---|---|
| BERLIN | 19-02-1987 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument